# EUROPEAN PATENT SPECIFICATION

(11) **EP 0 464 084 B1**
(45) Date of publication and mention of the grant of the patent: **09.07.1997**
(21) Application number: 90904918.1
(22) Date of filing: 20.03.1990
(51) Int. Cl.: A61K 31/20

(54) **USE OF EICOSAPENTAENOIC ACID FOR THE TREATMENT OF CACHEXIA**
VERWENDUNG VON EICOSAPENTAENSÄURE ZUR BEHANDLUNG DER KACHEXIE
EMPLOI D'ACIDE EICOSAPENTAENO QUE DANS LE TRAITEMENT DE LA CACHEXIE

(30) Priority: 20.03.1989 GB 8906369
(43) Date of publication of application: 08.01.1992
(73) Proprietor: SCOTIA HOLDINGS PLC, GB-London EC3N 4BB (GB)
(72) Inventor: TISDALE, Michael, John, Claverdon Warwickschire CV35 8LW (GB); BECK, Susan, Anne, West Midland WV12 5TE (GB)
(74) Representative: Skerrett, John Norton Haigh
(86) International application number: GB9000425
(87) International publication number: WO9011073

(56) References cited:
- EP-A- 378 824
- EP-A- 0 175 468
- EP-A- 0 335 550
- BIOCHIMICA ET BIOPHYSICA ACTA, vol. 889, 1986, Elsevier Science Publishers B.V.; K. LAUSTIOLA et al.: "Altered physio-logical responsiveness and decreased cyclic AMP levels in rat atria dietary cod liver oil supplementation and its possible association with an increased membrane phospholipid n - 3/n - 6 fatty acid ration", pp. 95-102
- J.N.C.J., vol. 73, no. 2, August 1984; R.A. KARMALL et al.: "Effect of omega-3 fatte acids on growth of a rat mammary tumor", pp. 457-461
- PROSTAGLANDINS LEUKOTRIENES AND MEDICINE, vol. 19, 1985; M.R. BEGIN et al.: "Selective killing of human cancer cells by polyunsaturated fatty acids", pp. 177-186
- IRCS MEDICAL SCIENCE, vol. 14, no. 4, 1986; J. BOOYENS et al.: "Suppression of proliferation of human larynx carcinoma cells in culture by gamma-linolenic and eicosapentaenoic acids with stimulation by oleic acid", p. 396
- J. ENZYM INHIB., vol. 1, no. 2, 1987; F.S. STEVEN et al.: "Further inhibition studies on guanidinobenzoatase, a trypsin-like enzyme associated with tumour cells", pp. 187-201

## Description

This invention relates to the omega-3 polyunsaturated fatty acid 5,8,11,14,17-eicosapentaenoic acid (herein referred to shortly as EPA, which term is to be understood as including also physiologically functional derivatives thereof, e.g. salts or esters), and to use in medicine of this compound for providing an active therapeutic agent. EPA is known to occur naturally as one of the several fatty acid constituents of marine oil, commonly called "fish oil".

### BACKGROUND

The present invention has arisen out of experimental studies investigating a newly-identified biologically active substance having a high lipolytic activity which seems to be specifically associated with a range of malignant tumours, especially cachexia-inducing tumours, in animals and in humans, as disclosed for example in the specification of our European patent application No. 89302740.9 (EU0335550). During the course of these studies, EPA was found to be an effective antagonist or inhibitor of this so-called lipolytic factor; it was also found to be effective in depressing an abnormal elevated level of cyclic adenylic acid (cAMP) produced in adipose tissue cells (adipocytes) by this so-called lipolytic factor or by other known lipolytically active substances, as evidenced by experiments conducted using mouse adipose tissue cell preparations. These are newly-identified properties of EPA not hitherto reported before the priority date of the present application.

### SUMMARY OF THE INVENTION

The present invention is based on the above findings and also on the further finding that administration of EPA is effective in vivo in suppressing the symptoms of cachexia, especially cancer cachexia, whereby it can provide a useful active therapeutic agent for treatment of this condition. This effect, it is believed, is related at least in part to in vivo activity peculiar to EPA in inhibiting the aforesaid newly-identified lipolytic factor and in reducing abnormal elevated cAMP levels in adipose tissue cells produced by this or by other lipolytic agents. It may also be related to inhibition of proteolytic activity which has been noted in respect of skeletal muscle in some instances of tumour growth.

It has also been found that EPA can have an inhibitory effect on guanidinobenzoatase which, it is believed, promotes an effect of EPA in reducing invasive and metastatic activities of malignant tumour cells.

Thus, according to one aspect of the present invention, 5,8,11,14,17-eicosapentaenoic acid or a physiologically functional derivative thereof, e.g. a salt or ester, herein collectively designated EPA, is used to make a medical preparation or medicament for therapeutic use in treating or preventing cachexia associated with lipolytic or fat mobilising substances present in body fluids in mammals, wherein the medical preparation or medicament contains an effective anti-cachectic amount of the EPA, not in the form of fish oil and not in the form of a food preparation but in the form of a pharmaceutical unit dosage for oral, parenteral or topical administration.

Also, according to the invention, EPA as defined above is used to make a medical preparation or medicament other than a food preparation for therapeutic treatment of mammals to reduce abnormal elevated cAMP levels detected in cells of their adipose tissue resulting from lipolytic or fat mobilising substances present in body fluids of said mammals, thereby to prevent the development of cachexia associated with such abnormal elevated cAMP levels and lipolytic or fat mobilising substances.

The invention also comprises the use hereinafter claimed of EPA as defined above for the manufacture of a medical preparation or medicament other than a food preparation for therapeutic treatment of mammals to inhibit abnormal lipolytic activity produced by biologically active lipolytic agents present in body fluids of said mammals, thereby to prevent the development of cachexia associated with such abnormal lipolytic activity.

The invention also comprises the use of EPA as defined above for the manufacture of a medical preparation or medicament other than a food preparation for therapeutic treatment to inhibit activity of the enzyme guanidinobenzoatase in malignant tumour bearing mammals and thereby specifically control tumour metastasis.

In carrying out the invention, in general an effective anti-cachectic and/or inhibiting amount of the EPA, or an effective amount of EPA for reducing an abnormal elevated level of cAMP in adipose tissue cells produced by lipolytic or fat mobilizing substance present in the body fluids or circulatory system of the mammal to be treated, will be made up as a non-dietary pharmaceutical formulation in unit dosage form ready for administration orally, parenterally (including subcutaneously, intramuscularly and intravenously), or topically. Such formulations may comprise a pharmaceutical composition, prepared by any of the methods well known in the art of pharmacy, in which the active EPA component is in intimate association or admixture with at least one other ingredient providing a compatible pharmaceutically acceptable carrier, diluent or excipient. Alternatively, such formulations may comprise a protective envelope of compatible or relatively inert pharmaceutically acceptable material within which is contained the active EPA component in the form of concentrated or pure EPA without association or admixture with any other ingredients.

Formulations of the present invention suitable for oral administration may be presented as discrete units such as capsules, cachets, tablets or lozenges, each containing a predetermined amount of the active EPA component, with capsules being a preferred type of formulation for providing the most effective means of oral delivery. For parenteral administration the formulations may comprise sterile liquid preparations of a predetermined amount of the active EPA component contained in sealed ampoules ready for use.

In a further alternative definition the invention, from one aspect, may also be expressed as providing a composition for inhibiting lipolytic activity of biologically active lipolytic agents present in body fluids in mammals and/or for inhibiting the enzyme guanidinobenzoatase associated with tumour cells and with cells capable of migration in mammals, characterised in that the composition comprises an effective inhibiting amount of EPA (as herein defined) together with a compatible pharmaceutically acceptable material or carrier.

Preferably, the EPA used for making the medical preparations, medicaments or compositions in accordance with the invention is of at least about 90% purity and will contain no more than minimal or pharmaceutically insignificant amounts of any other polyunsaturated fatty acids. A purity of more than 90% is recommended with the highest commercially available grade (about 95% purity), which is substantially free of any other polyunsaturated fatty acids, being the most preferred material.

Although EPA of high purity readily oxidises and is an inherently unstable compound under normal ambient conditions in the presence of air such that it usually requires to be stored out of sunlight at a low temperature under an atmosphere of nitrogen, difficulties in handling can be minimized by observing precautionary measures well known in the art. It will normally be protected from contact with air and sunlight in the pharmaceutical formulations into which it is made up for therapeutic use and such formulations may be kept stored at low temperatures until required for use.

By way of further background explanation and description of the invention, illustrative examples are hereinafter presented of investigations made and results obtained in the development of the invention, from which the skilled person in the art will more readily be able to appreciate the nature thereof and to put the invention into practical effect.

### BRIEF DESCRIPTION OF THE DRAWINGS

In the accompanying drawings,
FIGURES 1 and 2 are diagrams showing the effect of EPA on lipolytic activity associated with MAC16 tumour extracts;
FIGURE 3 is a diagram showing the effect of EPA on proteolytic activity associated with MAC16 tumour extracts;
FIGURE 4 is a bar chart diagram showing the effect of EPA on the activity of several different lipolytic agents;
FIGURE 5 is a similar bar chart diagram showing the effect of EPA on the level of cAMP in fat cells or adipocytes during incubation with the different lipolytic agents referred to in FIGURE 4;
FIGURE 6 is a diagram showing the inhibition effect of EPA on guanidinobenzoatase;
FIGURE 7 is a diagram showing the effect of oral dosing with EPA on the growth of MAC16 adenocarcinoma in female NMRI mice;
FIGURE 8 is a similar diagram showing the effect of oral dosing with EPA on body weight of female NMRI mice bearing the MAC16 tumour; and
FIGURES 9 and 10 are further diagrams similar to FIGURES 7 and 8 showing additional comparative results.

### MORE DETAILED DESCRIPTION

In some preliminary experiments using mice, evidence was first obtained suggesting that EPA could inhibit the growth in vivo of at least two different tumours, designated MAC16 and MAC13 respectively, belonging to an established series (MAC) of chemically induced, transplantable colon adenocarcinomas (see for example Cowen et al (1980), JNCI, 64, 675-681), and could reduce the weight loss or cachexia associated with the MAC16 tumour. The MAC16 tumour is a cachexia-inducing tumour associated with high levels of lipolytic activity and also with proteolytic activity; the MAC13 is (at least in mice) a non-cachexia inducing colon adenocarcinoma (albeit extracts thereof do show a certain level of lipolytic activity). Subsequent studies in vivo with the MAC16 tumour further indicated that the reduction in weight loss and inhibition of the tumour-associated lipolytic activity referred to above, as well as the tumour growth inhibition, was a dose related effect. However, the anticachectic effect of EPA appears, somewhat surprisingly, to exceed the antitumour effect.

During the course of continuing investigations of the newly identified so-called lipolytic factor, derived from the above-mentioned tumours as disclosed in our aforesaid European patent application No. 89302740.9 (EU0335550), a series of in vitro experiments was conducted to screen a range of various compounds, including EPA, for possible activity as inhibitors or antagonists to the lipolytic factor. In general, in these experiments the compounds to be tested were added to extracts from MAC16 tumours and incubated with freshly prepared adipocytes from mouse epididymal adipose tissue for 2 hrs. The lipolytic activity, or reduction thereof, was then determined by measuring the glycerol release using an enzyme assay that results in a production of NAD (nicotinamide adenine dinucleotide) from the reduced form NADH, the amount of NAD corresponding to the amount of glycerol present. The NAD was measured spectrophotometrically as a decrease in absorption at 340nm.

More specific details of the experimental procedures in these inhibition studies are summarised below:
1. Preparation of extracts from MAC16 tumours.
   MAC16 tumours from NMRI mice that had lost up to one third of their original body weight, were homogenised in Krebs-Ringer buffer at a concentration of 0.2g/ml. The homogenate was then centrifuged and the supernatant used for inhibition studies.
2. Preparation of adipocytes
   Fat pads were removed from 2 mice for the assay of each batch of 10 samples. 1 ml of collagenase solution in Krebs buffer (2 mg/ml) was added to the fat pads from 1 mouse which were then finely chopped prior to incubation for 2 hr at 37°C. After 2 hr the adipocytes were pooled, washed three times in Krebs buffer, and then counted to obtain a concentration of 1.5 - 2.0 x 10⁵ adipocytes per ml.
3. The experiment was set up as follows.
   100µl tumour extract + 1 ml fat cells
   Compound to be screened + 1 ml fat cells
   100µl tumour and compound + 1 ml fat cells
   Each compound was tested at increasing concentrations and all samples were prepared and processed in duplicate.
   The samples were gassed for 2 min with 95% O₂, 5% CO₂ mixture, mixed and incubated for 2 hr at 37°C. After 2 hr, 0.5 ml from each sample was then assayed for glycerol content.

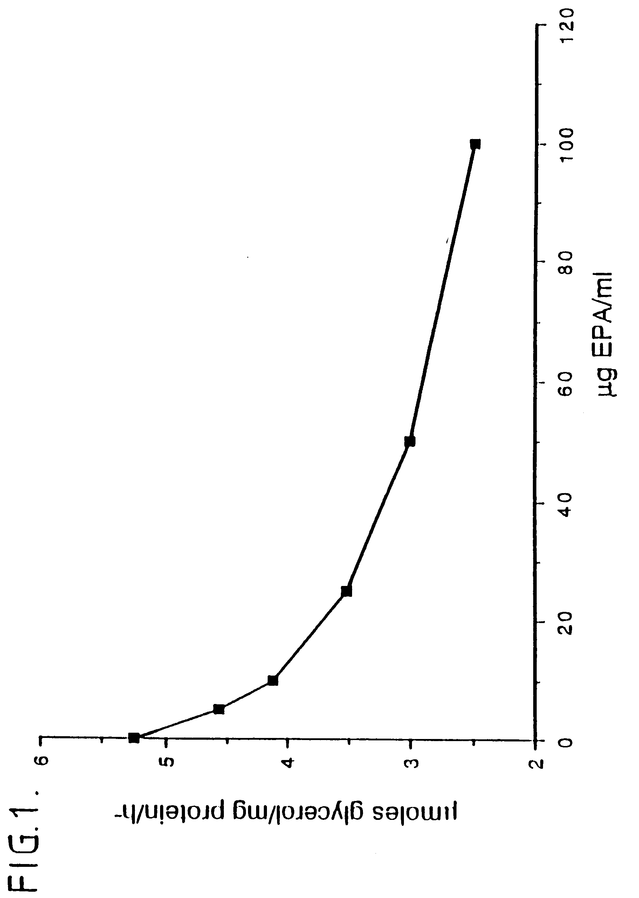

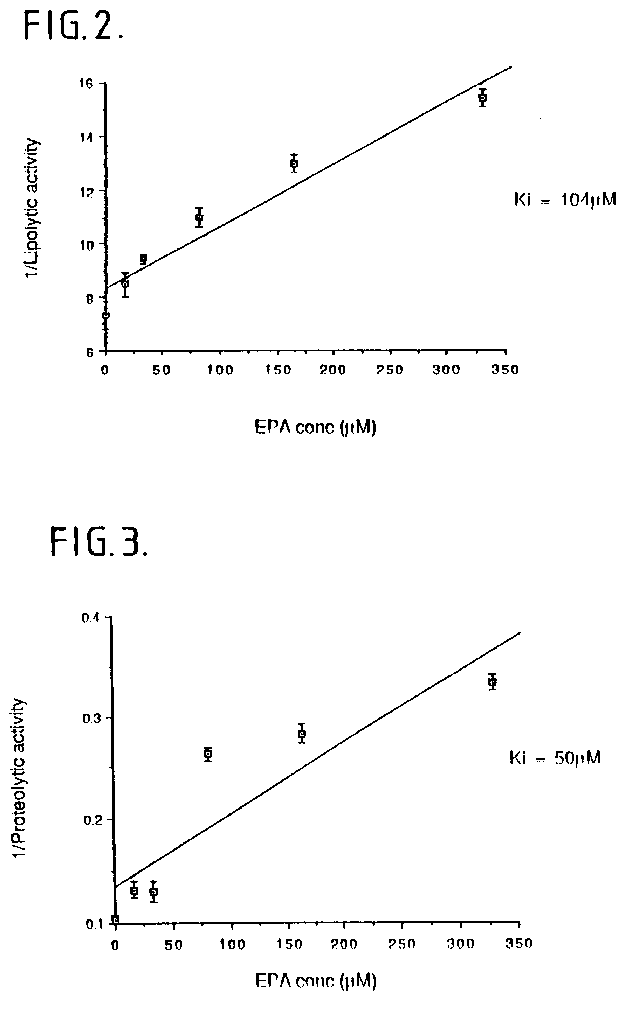

These experiments confirmed that EPA at a sufficient dosage has a strong inhibitory effect on the lipolytic activity of the lipolytic factor in the tumour extracts, as illustrated for example by FIGURE 1 of the accompanying drawings. This diagram shows the results obtained using EPA in one set of the above experiments and it also clearly indicates the dose dependence nature of the effect. In FIGURE 2, the same results are presented as a Dixon plot. Lipolytic activity is expressed as µmol glycerol released from the murine epididymal adipocytes per mg protein per hr. Results are expressed as mean ± S.E.M and the number of experiments performed was 3 to 4.

Similar tests on a range of compounds, including other related polyunsaturated fatty acids, failed to indicate any significant inhibition of the lipolytic factor in the tumour extracts by Linolenic acid, Octadocatetraenoic acid, Trans-3-Hexenoic acid, Trans 2-Hexenoic acid, Cis-3-Hexen-1-o1, 3-Octenoic acid, Linoleic acid, Eicosatrienoic acid, Arachidonic acid, Palmitoleic acid, Nicotinic acid, Adenosine and Inosine. As will hereinafter appear, docosahexaenoic acid also appears not be act as an inhibitor of the activity of this tumour lipid mobilising agent or lipolytic factor.

In addition, in similar experiments wherein extracts of MAC16 tumours were incubated with mouse diaphragm, EPA was found to inhibit the proteolytic activity, as shown for example in FIGURE 3 in which the results of typical experiments are again presented as a Dixon plot. In this diagram, proteolytic activity of the tumour extracts is expressed in terms of nmoles total amino acid released per gram diaphragm per mg protein per 2hr and are corrected for spontaneous amino acid release. Results are expressed as mean ± S.E.M and the number of experiments performed was 3 to 4.

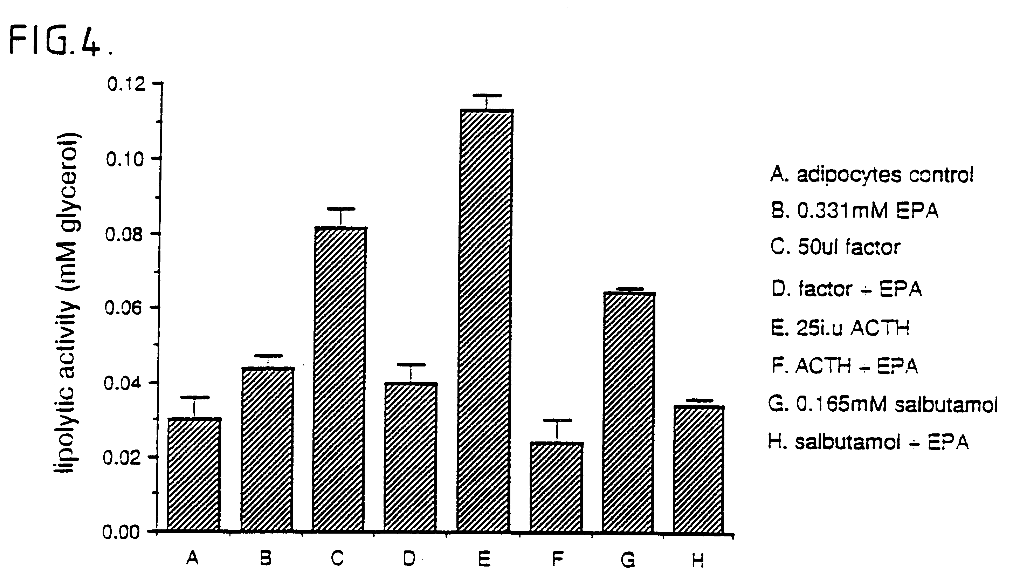

In a further series of similar experiments, EPA was found to act as an effective inhibitor not only of the lipolytic factor from the tumour extracts referred to above, but also of some other substances that are known to function as biologically active lipolytic agents. These included ACTH (the lipase activating adrenocorticotropic hormone) and Salbutamol, the effects being illustrated in FIGURE 4 of the accompanying drawings which is a bar chart diagram showing the relative levels of lipolytic activity detected under comparable conditions for the various test samples enumerated.

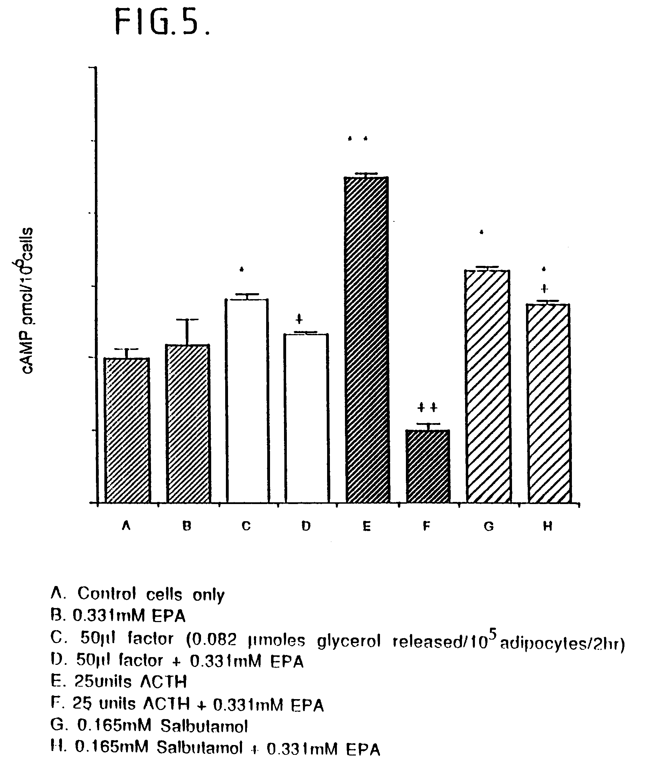

In the course of these latter experiments, tests were also carried out to measure (by known techniques) the level of cyclic adenylic acid (cAMP) in the fat cells or adipocytes during the period of incubation with the various lipolytic agents, both in the presence of and without the presence of EPA. The results, after a 10 minute incubation period, are presented in the bar chart diagram of FIGURE 5 of the accompanying drawings which should be compared with FIGURE 4. The results in FIGURE 5 are expressed as the Mean ± S.E.M of 3 to 4 experiments. It will be seen that each of the three specific lipolytic agents tested, when present on its own and lipolytically most active, gives rise to an abnormal elevated level of cAMP in the cells, but this elevated level is depressed in the presence of EPA. This suggests that normally, like many hormones, these lipolytic agents bind to specific receptor sites on the membrane of the fat cells or adipocytes concerned and act to bring about a modification of the intracellular level of cAMP which is known to act as a so-called secondary hormone that regulates the activity of enzyme systems within the cell. The indications are that the EPA interferes with and inhibits this effect, acting specifically somewhere in the adenylate cyclase cascade to inhibit the production of cAMP in response to lipolytic stimuli, perhaps by itself binding to and blocking the receptor sites involved. Again, this property has been found to be unique to EPA and is not shown by other polyunsaturated fatty acids.

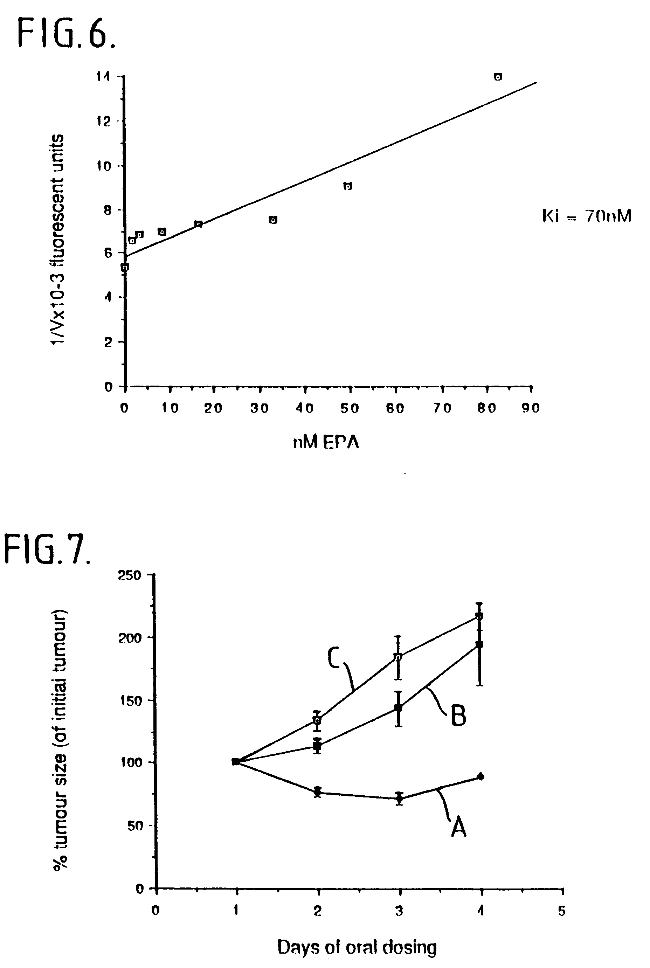

In addition, as illustrated by the Dixon plot of results shown in FIGURE 6, EPA has been found to be a highly effective inhibitor of the enzyme guanidinobenzoatase which is a trypsin-like enzyme associated with tumour cells and cells capable of migration and which, it has been suggested, may play a role in tumour metastasis. In deriving the results shown in FIGURE 6, enzyme activity was determined by the release of the fluorogenic product methyl umbelliferone from 4-methylumbelliferyl-p-guanidinobenzoate by 1µl of NMRI mouse serum. The inhibitor was added to the enzyme together with the substrate and the decrease of fluorescence at 446nm was determined. In this property EPA is again also unique in that it appears to be the only fatty acid capable of effectively inhibiting guanidinobenzoatase.

In continuing in vivo experiments, it has been found that the pure EPA is surprisingly more effective as a therapeutic agent than had been anticipated and also it appears to have a very low toxicity. We have compared oral dosing of 95% pure EPA in the form of the triglyceride ester (obtained from Peninsula Laboratories, Merseyside, United Kingdom) with linoleic acid in mice bearing the MAC16 adenocarcinoma at a dose of 5g kg⁻¹. No toxicity or other adverse effects were noted and the results in FIGURE 7 show EPA at this dose level to produce an extensive growth delay. In obtaining these results, mice (20g weight) were dosed orally with either 100µl EPA/day/mouse (A), 100µl linoleic acid/day/mouse (B) or 100µl 0.9% saline/day/mouse (C). The experiment was initiated 14 days after tumour transplantation when the tumours became palpable (average initial tumour volume = 176±23 mm³). Tumour volumes were measured daily and recorded as a percentage of the tumour volume prior to oral dosing. Results are expressed as the mean ± S.E.M.

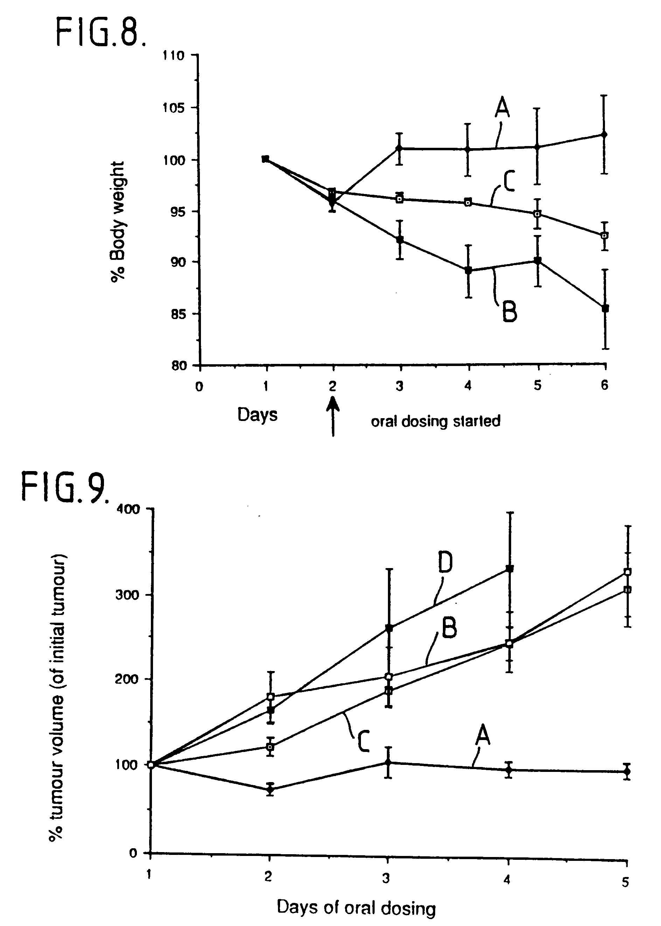

The effect of the fatty acids on host body weight is shown in FIGURE 8. Again mice (20g) were dosed orally with either 100µ EPA/day/mouse (A), 100µl linoleic acid/day/mouse (B) or 100µl 0.9% saline/day/mouse (C). Treatment was initiated 14 days after tumour transplantation when weight loss became apparent (average weight loss 5%). In this case, body weights were measured daily and recorded as a percentage of the body weight prior to oral dosing, the results again being expressed as the mean ± S.E.M. There was no significant difference in food and water intake. As will be seen, the results show EPA also effectively blocked the cachectic effect of the tumour whereas linoleic acid at an equivalent dose level had an enhancing effect.

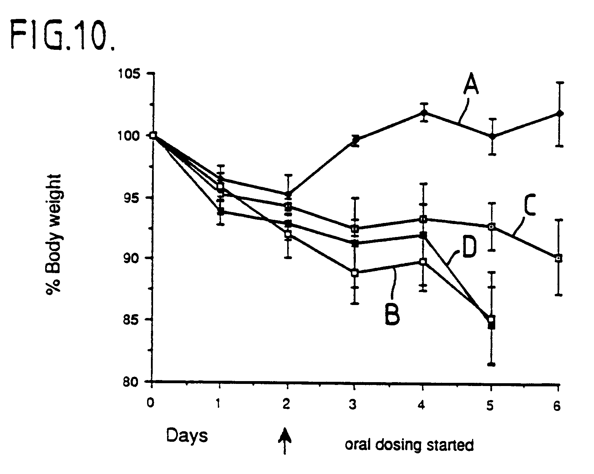

In further experiments, of which the results are shown in FIGURES 9 and 10, the effect of dosing with pure EPA was also compared with the effect of linoleic acid and with that of docosahexaenoic acid (purchased from Sigma Chemical Co., Poole, U.K.), herein referred to shortly as DHA.

FIGURES 9 and 10 shows the results of experiments in which again mice (20g) were used and dosed orally with either 100µl EPA/day/mouse (A), 100µl linoleic acid/day/mouse (B), 100µl 0.9% saline/day/mouse (C) or 100µl DHA/day/mouse (D).

The experiment of which the results are shown in FIGURE 9 was initiated 14 days after tumour transplantation when the tumours became palpable (average initial tumour volume = 96±12mm³). Tumour volumes were measured daily and recorded as a percentage of the tumour volume prior to oral dosing. Results are expressed as the mean ± S.E.M.

The effect on host body weight from the treatment relating to FIGURE 9 is shown in FIGURE 10. The treatment was initiated 14 days after tumour transplanation when weight loss became apparent (average weight loss 7%). Body weights were measured daily and recorded as a percentage of the body weight prior to oral dosing. Results are expressed as the mean ± S.E.M. There was no significant difference in food and water intake.

The results presented in these FIGURES 9 and 10 demonstrate clearly that only EPA possesses antitumour and anticachectic activity. In contrast, the related omega-3 polyunsaturated fatty acid DHA, which is also a major constituent of fish oil (18.7%) and which differs from EPA only in the presence of two extra carbon atoms and another double bond, was totally devoid of any antitumour or anticachectic activity, when administered orally at the same dose as EPA (5g kg⁻¹). Moreover, while EPA was non-toxic at this dose level, DHA showed marked signs of toxicity, as evidenced by an increased weight loss compared with the control, and the experiments had to be terminated after only a few oral doses. It has also been ascertained that administration of EPA significantly reduces the level of arachidonic acid (ARA) which appears in the blood plasma of animals bearing the MAC16 tumour However, a similar effect has also been found with administration of DHA, and since only EPA exerts antitumour and anticachectic activity against the MAC16 tumour, this indicates that reductions in tumour ARA, leading perhaps to an inhibition of prostaglandin synthesis, at least in themselves are not of prime importance for either activity.

These results further confirm that in respect of such antitumour and anticachectic effects as have been found, not only is pure EPA even more efficacious and less toxic than anticipated, but EPA appears to have special properties not shared by the other polyunsaturated fatty acids.

Thus, the inhibiting or antagonistic effects found for EPA against lipolytic agents that are known to be physiologically active and often associated with a condition of cachexia and also with tumour growth, such as the newly-identified lipolytic factor produced by MAC16 adenocarcinoma tumours, have demonstrated an unexpected high potential for EPA as a valuable therapeutic agent, especially in the treatment of cachexia and/or tumours in mammals, particularly as such newly-identified lipolytic factor is now known to be associated with many other tumours including, it is believed, many if not most human tumours.

## Claims

1. Use of 5,8,11,14,17-eicosapentaenoic acid or a physiologically functional derivative thereof (herein collectively designated EPA) for the manufacture of a medical preparation or medicament for therapeutic use in treating or preventing cachexia associated with lipolytic or fat mobilising substances present in body fluids in mammals, wherein the medical preparation or medicament contains an effective anti-cachectic amount of the EPA, not in the form of fish oil and not in the form of a food preparation but in the form of a pharmaceutical unit dosage for oral, parenteral or topical administration.

2. Use of 5,8,11,14,17-eicosapentaenoic acid or a physiologically functional derivative thereof (herein collectively designated EPA) for the manufacture of a medical preparation or medicament other than a food preparation for therapeutic treatment of mammals to reduce abnormal elevated cAMP levels detected in cells of their adipose tissue resulting from lipolytic or fat mobilising substances present in body fluids of said mammals, thereby to prevent the development of cachexia associated with such abnormal elevated cAMP levels and lipolytic or fat mobilising substances.

3. Use of EPA as claimed in Claim 2 wherein an amount of the EPA that is effective to reduce said abnormal elevated level of cAMP in adipose tissue cells of the mammal to be treated is incorporated in the medical preparation or medicament which is made up as a pharmaceutical formulation in unit dosage form.

4. Use of 5,8,11,14,17-eicosapentaenoic acid or a physiologically functional derivative thereof (herein collectively designated EPA) for the manufacture of a medical preparation or medicament other than a food preparation for therapeutic treatment of mammals to inhibit abnormal lipolytic activity produced by biologically active lipolytic agents present in body fluids of said mammals, thereby to prevent the development of cachexia associated with such abnormal lipolytic activity, wherein an effective lipolytic agent inhibiting amount of the EPA, not in the form of fish oil, is incorporated in the medical preparation or medicament which is made up as a non-dietary pharmaceutical formulation in unit dosage form.

5. Use of 5,8,11,14,17-eicosapentaenoic acid or a physiologically functional derivative thereof (herein collectively designated EPA) for the manufacture of a medical preparation or medicament other than a food preparation for therapeutic treatment to inhibit activity of the enzyme guanidinobenzoatase in malignant tumour bearing mammals and thereby specifically control tumour metastasis, wherein an effective guanidinobenzoatase inhibiting amount of the EPA is incorporated in the medical preparation or medicament which is made up as a pharmaceutical formulation in unit dosage form.

6. Use of EPA as claimed in any one of Claims 1 to 5, wherein the EPA is of at least 90% purity.

7. Use of EPA as claimed in any one of Claims 1 to 5 wherein the EPA is about 95% pure.

8. Use of EPA as claimed in any one of Claims 1 to 7, wherein the EPA is substantially free of any other polyunsaturated fatty acids, the latter if present being in no more than minimal or pharmaceutically insignificant amounts.

9. Use of EPA as claimed in any one of the preceding claims wherein the EPA contained in said medical preparation or medicament is protected from contact with air and sunlight.

## Patentansprüche

1. Verwendung einer 5, 8, 11, 14, 17-Eicosapentaensäure oder deren physiologisch funktionellen Derivativs (hier zusammenfassend EPA bezeichnet) zur Herstellung eines medizinischen Präparats oder Medikaments für therapeutische Zwecke zum Behandeln oder Verhüten einer Kachexie, welche mit lipolythischen oder fettmobilisierenden, in Körperflüssigkeiten von Säugern vorherrschenden Substanzen verbunden ist, wobei das medizinische Präparat oder Medikament eine wirksame antikachektische Menge an EPA nicht in Form von Fischöl und nicht in Form einer Nahrungsmittelzubereitung, sondern in Form einer pharmazeutischen Einheitsdosierung für eine orale, parenterale oder topische Verabreichung enthält.

2. Verwendung einer 5, 8, 11, 14, 17-Eicosapentaensäure oder deren physiologisch funktionellen Derivativs (hier zusammenfassend EPA bezeichnet) zur Herstellung eines medizinischen Präparates oder Medikaments, nicht jedoch einer Nahrungsmittelzubereitung, zur therapeutischen Behandlung von Säugern zwecks Verringerung abnorm erhöhter cAMP-Werte, welche in Zellen von deren Fettgewebe festgestellt werden und von lipolytischen oder fettmobilisierenden, in Körperflüssigkeiten der Säuger auftretenden Substanzen herrühren, um dadurch die Entwicklung der Kachexie zu verhindern, welche mit derartig abnorm erhöhten cAMP-Werten und den lipolytischen oder fettmobilisierenden Substanzen verbunden ist.

3. Verwendung von EPA nach Anspruch 2, dadurch gekennzeichnet, daß eine Menge an EPA, welche in bezug auf eine Reduzierung des abnorm erhöhten cAMP-Wertes in Fettgewebezellen des zu behandelnden Säugers wirksam ist, in das medizinische Präparat oder Medikament eingearbeitet wird, welches als pharmazeutische Zubereitung in Einheitsdosierungsform bereitgestellt wird.

4. Verwendung einer 5, 8, 11, 14, 17-Eicosapentaensäure oder deren physiologisch funktionellen Derivativs (hier zusammenfassend EPA bezeichnet) zur Herstellung eines medizinischen Präparats oder Medikaments, nicht jedoch einer Nahrungsmittelzubereitung, zur therapeutischen Behandlung von Säugern, um abnorme lipolytische Aktivitäten, welche durch biologisch aktive, lipolytische, in Körpenflüssigkeiten der Säuger auftretende Mittel erzeugt wird, zu verhindern, um dadurch die Entwicklung der Kachexie, welche mit einer derart abnormen lipolytischen Aktivität verbunden ist, zu verhindern, wobei eine wirksame, das lipolytische Mittel verhindende Menge an EPA nicht in Form von Fischöl in das medizinische Präparat oder Medikament eingearbeitet wird, welches als nichtdiätetische pharmazeutische Zubereitung in Einheitsdosierungsform bereitgestellt wird.

5. Verwendung einer 5, 8, 11, 14, 17-Eicososapentaensäure oder deren physiologisch funktionellen Derivativs (hier zusammenfassend EPA bezeichnet) zur Herstellung eines medizinischen Präparats oder Medikaments, nicht jedoch einer Nahrungsmittelzubereitung, zur therapeutischen Behandlung zwecks Inhibierung der Aktivität des Enzyms Guanidinobenzoatase in einen bösartigen Tumor tragenden Säugern und spezifischer Überwachung von Tumormetastasen, wobei eine wirksame, Guanidinobenzoatase verhindernde Menge an EPA in das medizinische Präparat oder Medikament eingearbeitet wird, welches als pharmazeutische Zubereitung in Einheitsdosierungsform bereitgestellt wird.

6. Verwendung von EPA nach einem der Ansprüche 1 bis 5, dadurch gekennzeichnet, daß die EPA eine Reinheit von wenigstens 90% aufweist.

7. Verwendung von EPA nach einem der Ansprüche 1 bis 5, dadurch gekennzeichnet, daß die EPA eine Reinheit von etwa 95 % aufweist.

8. Verwendung von EPA nach einem der Ansprüche 1 bis 7, dadurch gekennzeichnet, daß die EPA und wesentlichen frei von anderen mehrfach ungesättigten Fettsäuren ist, wobei die letzteren, falls sie vorliegen, höchstens in minimalen oder pharmazeutisch unbedeutenden Mengen auftreten.

9. Verwendung von EPA nach einem der vorhergehenden Ansprüche, dadurch gekennzeichnet, daß das in der medizinischen Zubereitung oder dem Medikament enthaltene EPA vor einem Kontakt mit Luft und Sonnenlicht geschützt ist.

## Revendications

1. Utilisation de l'acide 5,8,11,14,17-eicosapentaénoïque ou d'un dérivé physiologiquement fonctionnel de celui-ci (collectivement dénommés ici EPA) pour la fabrication d'une préparation médicale ou d'un médicament pour utilisation thérapeutique dans le traitement ou la prévention de la cachexie associée avec des substances lipolytiques ou mobilisant la graisse présentes dans les fluides corporels de mammifères, dans laquelle la préparation médicale ou le médicament contient une quantité anticachectique efficace de l'EPA, pas sous la forme d'huile de poisson ni sous la forme d'une préparation alimentaire, mais sous la forme d'une unité galénique pharmaceutique pour administration orale, parentérale ou topique.

2. Utilisation de l'acide 5,8,11,14,17-eicosapentaénoïque ou d'un dérivé physiologiquement fonctionnel de celui-ci (collectivement dénommés ici EPA) pour la fabrication d'une préparation médicale ou d'un médicament autre qu'une préparation alimentaire pour le traitement thérapeutique de mammifères pour réduire des taux d'AMPc élevés anormaux détectés dans des cellules de leur tissu adipeux, résultant de substances lipolytiques ou mobilisant la graisse présentes dans les fluides corporels desdits mammifères, pour prévenir ainsi le développement de cachexie associée avec de tels taux d'AMPc élevés anormaux et des substances lipolytiques ou mobilisant la graisse.

3. Utilisation d'EPA selon la revendication 2, dans laquelle une quantité de l'EPA qui est efficace pour réduire ledit taux d'AMPc élevé anormal dans des cellules de tissu adipeux du mammifère à traiter est incorporée dans la préparation médicale ou le médicament qui est façonné(e) sous la forme d'une formulation pharmaceutique en forme galénique unitaire.

4. Utilisation de l'acide 5,8,11,14,17-eicosapentaénoïque ou d'un dérivé physiologiquement fonctionnel de celui-ci (collectivement dénommés ici EPA) pour la fabrication d'une préparation médicale ou d'un médicament autre qu'une préparation alimentaire pour le traitement thérapeutique de mammifères pour inhiber une activité lipolytique anormale produite par des agents lipolytiques biologiquement actifs présents dans les fluides corporels desdits mammifères, pour prévenir ainsi le développement de cachexie associée avec une telle activité lipolytique anormale, dans laquelle une quantité efficace de l'EPA inhibant un agent lipolytique, pas sous la forme d'une huile de poisson, est incorporée dans la préparation médicale ou le médicament qui est façonné(e) sous la forme d'une formulation pharmaceutique non alimentaire en forme galénique unitaire.

5. Utilisation de l'acide 5,8,11,14,17-eicosapentaénoïque ou d'un dérivé physiologiquement fonctionnel de celui-ci (collectivement dénommés ici EPA) pour la fabrication d'une préparation médicale ou d'un médicament autre qu'une préparation alimentaire pour le traitement thérapeutique pour inhiber l'activité de l'enzyme guanidinobenzoatase chez des mammifères portant une tumeur maligne et maîtriser ainsi spécifiquement la métastase de tumeur, dans laquelle une quantité efficace de l'EPA inhibant la guanidinobenzoatase est incorporée dans la préparation médicale ou le médicament qui est façonné(e) sous la forme d'une formulation pharmaceutique en forme galénique unitaire.

6. Utilisation d'EPA selon l'une quelconque des revendications 1 à 5, dans laquelle l'EPA est d'une pureté d'au moins 90 %.

7. Utilisation d'EPA selon l'une quelconque des revendications 1 à 5, dans laquelle l'EPA est d'une pureté d'environ 95 %.

8. Utilisation d'EPA selon l'une quelconque des revendications 1 à 7, dans laquelle l'EPA est substantiellement exempt de tous autres acides gras polyinsaturés, ceux-ci, s'ils sont présents, étant dans des quantités pas plus que minimales ou bien pharmaceutiquement insignifiantes.

9. Utilisation d'EPA selon l'une quelconque des revendications précédentes, dans laquelle l'EPA contenu dans ladite préparation médicale ou ledit médicament est protégé du contact avec l'air et la lumière du soleil.
